# EUROPÄISCHE PATENTSCHRIFT

(11) **EP 1 742 746 B1**
(45) Veröffentlichungstag und Bekanntmachung des Hinweises auf die Patenterteilung: **23.07.2008**
(21) Anmeldenummer: 05740225.7
(22) Anmeldetag: 26.04.2005
(51) Int. Cl.: B05B 11/00

(54) **MANUELL BETÄTIGBARER SPENDER MIT SCHUTZKAPPE**
MANUALLY OPERATED DISPENSER COMPRISING A PROTECTIVE CAP
DISTRIBUTEUR A COMMANDE MANUELLE POURVU D'UN CAPUCHON PROTECTEUR

(30) Priorität: 03.05.2004 DE 102004021668
(43) Veröffentlichungstag der Anmeldung: 17.01.2007
(73) Patentinhaber: MeadWestvaco Calmar GmbH, 58675 Hemer (DE)
(72) Erfinder: WELP, Gisbert, 59846 Sundern (DE)
(74) Vertreter: Henseler, Daniela
(86) Internationale Anmeldenummer: PCT/EP2005/004453
(87) Internationale Veröffentlichungsnummer: WO 2005/107955

(56) Entgegenhaltungen:
- US-A- 4 944 429
- US-A- 6 092 692
- US-B1- 6 173 868

## Beschreibung

Die Erfindung betrifft einen manuell betätigbaren Spender für fließfähige Medien mit einem eine Vorratskammer aufweisenden Behälter und einem daran befestigbaren Pumpenkopf nach dem Oberbegriff des Anspruchs 1.

Aus EP 1 132 143 A2 ist ein manuell betätigbarer Spender mit einer Schutzkappe bekannt, um einen Nasenadapter vor Verschmutzungen oder Beschädigung während des Nichtgebrauchs zu schützen. Die Schutzkappe besteht aus zwei Schutzkappenhälften, die als formangepaßte Flügel über Scharniere an dem stutzenförmigen Ausbringabschnitt des Nasenadapters befestigt sind. Im aufgeklappten Zustand bilden die Flügel Griffflächen zum Betätigen des Pumpenkopfes. Ein solcher Spender besitzt den Vorteil einer schlanken Bauform. Die Übertragung des Betätigungsdrucks über die Flügel auf den Pumpenkopf macht jedoch stabile Abstützelemente erforderlich, wodurch der Spender schwerer wird und damit zwangsläufig unhandlicher.

Aufgabe der Erfindung ist es daher, einen Spender nach dem Oberbegriff des Anspruchs 1 zu schaffen, der mit einer Schutzkappe versehen und dabei handlich ist.

Diese Aufgabe wird durch die Merkmale des kennzeichnenden Teils des Anspruchs 1 gelöst.

Hierdurch wird ein Spender geschaffen, der eine auf- und zuklappbare Schutzkappe aus zwei Schutzkappenhälften besitzt, die unverlierbar an dem Spenderkopf befestigt ist. Die Schutzkappenhälften, die als formangefaßte Flügel ausgebildet sind, werden über Filmscharniere an den Endkanten der Griffflächen mit dem Pumpenkopf verbunden. Die Verwendung von Filmscharnieren gewährt nicht nur eine hohe Lebensdauer, sondern schafft auch die Möglichkeit eine leichte Schutzkappe vorzusehen. Eine Anlenkung der Schutzkappenhälften an den Endkanten der Griffflächen erlaubt zudem eine Ausbildung der Schutzkappe als formangepaßte Überdeckung einer gesamten Kopfseite eines Spenders.

Vorteilhaft ist ferner, daß die abklappbare Schutzkappe keine wesentliche Gestaltungsänderung des Pumpenkopfs verlangt. Die Schutzkappe kann einfach angehängt werden. Der Ausbringabschnitt und die Schutzkappe können dabei sogar in einem Teil gespritzt werden

Die Endkanten der Griffflächen können zur Ausbildung von Band-Filmscharnieren mit einer wählbaren Längserstreckung ausgebildet werden. Der Abklappwinkel der Schutzkappenhälften kann mittels einer Rippe eingestellt werden. An den Schutzkappenhälften kann ferner ein Blockierkeil vorgesehen sein, der durch das Griffteil eintauchen und einen Arretierstift auslenken kann. Der mit einer zugeklappten Schutzkappe schützbare Pumpenkopf kann dann gleichzeitig auch blockierbar sein.

Weitere Ausgestaltungen der Erfindung sind der nachfolgenden Beschreibung und den Unteransprüchen zu entnehmen.

Die Erfindung wird nachstehend anhand des in den beigefügten Abbildungen dargestellten Ausführungsbeispiels näher erläutert.
Fig. 1 zeigt schematisch eine Seitenansicht eines Spenders mit geschlossener Schutzkappe,
Fig. 2 zeigt schematisch eine Seitenansicht des Spenders mit geöffneter Schutzkappe,
Fig. 3 zeigt schematisch eine Draufsicht des Spenders,
Fig. 4 zeigt schematisch eine Seitenansicht des Spenders mit geschlossener Schutzkappe mit einem Teilschnitt im Bereich der Blockiermittel.

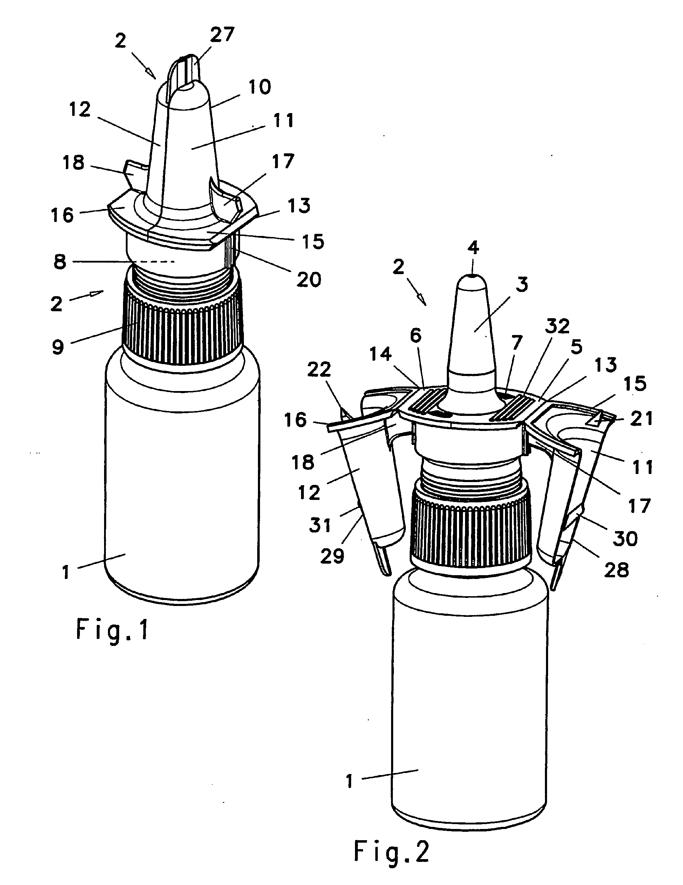

Die Figuren 1 und 2 zeigen einen manuell betätigbaren Spender für fließfähige Medien mit einem eine Vorratskammer aufweisenden Behälter 1 und einem daran befestigbaren Pumpenkopf 2. Der Pumpenkopf 2 umfaßt einen Ausbringabschnitt mit einem stutzenförmigen Nasenadapter 3, der kopfseitig eine als Zerstäuberdüse ausgebildete Austrittsöffnung 4 aufweist. Seitlich von dem Nasenadapter 3 abragende Griffflächen 5, 6 erstrecken sich auf gegenüberliegenden Seiten. Die Griffflächen 4, 5 können über einen mittigen Abschnitt 7 verbunden sein.

Der Nasenadapter 3 sitzt auf einem Kolben 8 einer Schubkolbenpumpe 9, die an dem Behälter 1 befestigt ist. Zum Ausbringen einer Charge in einem oder mehreren Hüben werden Nasenadapter 3 und Behälter 1 gegeneinander bewegt durch Auflage wenigstens eines Fingers auf den Griffflächen 5, 6.

Der Pumpenkopf 2 umfaßt ferner eine aus zwei Hälften bestehende Schutzkappe 10, die zwei aufklappbare formangepaßte Flügel 11, 12 aufweist. Zur Befestigung der Flügel 11, 12 am Pumpenkopf 2 weisen die Griffflächen 5, 6 an gegenüberliegenden Seiten Endkanten auf, die über jeweils ein Filmscharnier 13, 14 einstückig mit einem Flügel 11, 12 der Schutzkappe 10 verbunden sind. Die Flügel 11, 12 weisen dazu Bodenflächen 15, 16 auf, die die Griffflächen 5, 6 bei geschlossener Schutzkappe 10 überdecken.

Die Filmscharniere 13, 14 erlauben ein Aufklappen und Zuklappen der Flügel 11, 12, wodurch die Schutzkappe 10 geschlossen und geöffnet werden kann. Im geöffneten Zustand bleiben die Flügel 11, 12 mit dem Pumpenkopf 2 verbunden, so daß diese unverlierbar sind. Die Schutzkappe 10 ist vorzugsweise in einem Teil gespritzt mit den Grifffiächen 5, 6. Die Schutzkappe 10 kann dabei dünnwandig ausgebildet sein.

Die Filmscharniere 13, 14 erstrecken sich vorzugsweise über die gesamte Länge der Endkanten der Griffflächen 5, 6, um bandartige Filmscharniere auszubilden. Ferner wird hierdurch ein seitlicher Verschluß zur Überdeckung des Nasenadapters 3 einschließlich Griffflächen 5, 6 erreicht.

Jeder Flügel 11, 12 weist außenseitig mindestens eine quer zu den Filmscharnieren 13, 14 verlaufende Rippe 17, 18 auf, die jeweils eine Abstützfläche 19 besitzen, die bei aufgeklappten Flügeln 11, 12, wie in Fig. 2 dargestellt, mit einem Anschlag 20 am Pumpenkopf 2 in Eingriff bringbar ist.

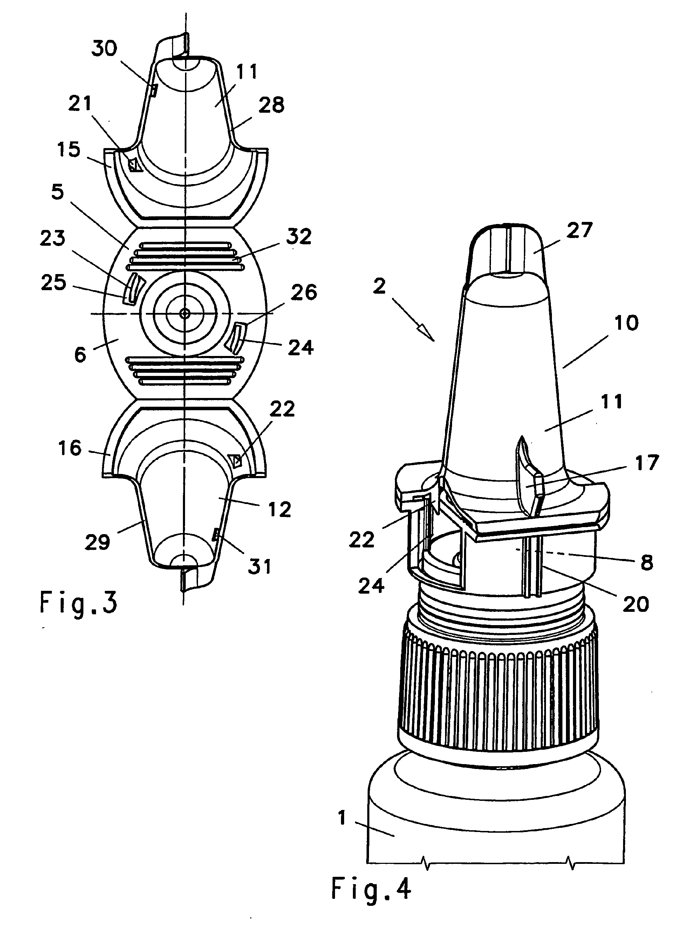

Jeder Flügel 11, 12 weist ferner vorzugsweise einen Blockierkeil 21, 22 auf, die bei zugeklappten Flügeln 11, 12, also geschlossener Schutzkappe 10, am Pumpenkopf 2 angeordnete Arretierstifte 23, 24 (vgl. Fig. 3 und 4) auslenken. Die Blockierkeile 21, 22 tauchen dazu durch Ausnehmungen 25, 26 in den Griffflächen 5, 6 ein.

Die Flügel 11, 12 können in dem zugeklappten Zustand fixbar sein, beispielsweise über einen seitlichen oder kopfseitigen Clipverschluß oder Schnappverschluß. Weiterhin können die Flügel 11, 12 mindestens einen gegenüber einer Flügelanlagefläche 28, 29 vorspringende Haltestifte 30, 31 aufweisen zum ausrichtenden Eingriff in den jeweils anderen Flügel 11, 12 bei geschlossener Schutzkappe 10.

Die Griffflächen 5, 6 können schließlich vorstehende Antirutschelemente 32 tragen, die bei geschlossener Schutzkappe 10 in Ausnehmungen im Bereich einer Bodenfläche der Flügel 11, 12 eintauchen können.

## Patentansprüche

1. Manuell betätigbarer Spender für fließfähige Medien mit einem eine Vorratskammer aufweisenden Behälter und einem daran befestigbaren Pumpenkopf, der einen Ausbringabschnitt mit einem stutzenförmigen, eine als Zerstäuberdüse ausgebildete Austrittsöffnung aufweisenden Nasenadapter und seitlich davon abragende Griffflächen aufweist, und mit einer zwei aufklappbare, formangepaßte Flügel aufweisenden Schutzkappe für den Nasenadapter, **dadurch gekennzeichnet, daß** die Griffflächen (5, 6) an gegenüberliegenden Seiten Endkanten aufweisen, die über jeweils ein Filmscharnier (13, 14) einstückig mit einem Flügel (11, 12) der Schutzkappe (10) verbunden sind und die Flügel (11, 12) Bodenflächen (15, 16) aufweisen, die die Griffflächen (5, 6) überdecken.

2. Manuell betätigbarer Spender nach Anspruch 1, **dadurch gekennzeichnet, daß** die Filmscharniere (13, 14) über die gesamte Länge der Endkanten der Griffflächen (5, 6) ausgebildet sind.

3. Manuell betätigbarer Spender nach Anspruch 1 oder 2, **dadurch gekennzeichnet, daß** die Flügel (11, 12) jeweils mindestens eine quer zu den Filmscharnieren (13, 14) verlaufende Rippe (17, 18) aufweisen, die eine Stützfläche (19) besitzt, die bei aufgeklappten Flügeln (11, 12) mit einem an dem Pumpenkopf (2) vorgesehenen Anschlag (20) in Eingriff bringbar ist.

4. Manuell betätigbarer Spender nach einem der Ansprüche 1 bis 3, **dadurch gekennzeichnet, daß** die Flügel (11, 12) jeweils einen Blockierkeil (21, 22) aufweisen, die bei zugeklappten Flügeln (11, 12) am Pumpenkopf (2) angeordnete Arretierstifte (23, 24) auslenken.

5. Manuell betätigbarer Spender nach einem der Ansprüche 1 bis 4, **dadurch gekennzeichnet, daß** die Flügel (11, 12) über einen Schnappverschluß (27) in einem zugeklappten Zustand fixierbar sind.

6. Manuell betätigbarer Spender nach einem der Ansprüche 1 bis 5, **dadurch gekennzeichnet, daß** die Flügel (11, 12) mindestens einen gegenüber einer Flügelanlagefläche (28, 29) vorspringenden Haltestift (30, 31) zum ausrichtenden Eingriff in den jeweils anderen Flügel (11, 12) aufweisen.

7. Manuell betätigbarer Spender nach einem der Ansprüche 1 bis 6, **dadurch gekennzeichnet, daß** die Griffflächen (5, 6) vorstehende Antirutschelemente (32) tragen, die bei geschlossener Schutzkappe (10) in Ausnehmungen im Bereich einer Bodenfläche der Flügel (11, 12) eintauchen.

## Claims

1. A manually operated dispenser for flowable media, having a container which has a supply chamber, and a pump head which can be fastened thereto and has a dispensing section with a nozzle-shaped nasal adapter having an outlet opening designed as a spray diffuser, and gripping surfaces protruding laterally from said nasal adapter, and having a protective cap, which has two wings which can be unfolded and are matched in terms of shape, for the nasal adapter, **characterized in that** the gripping surfaces (5, 6) have, on opposite sides, end edges which are connected as a single piece to a wing (11, 12) of the protective cap (10) via a respective film hinge (13, 14), and the wings (11, 12) have base surfaces (15, 16) which overlap the gripping surfaces (5, 6).

2. The manually operated dispenser as claimed in claim 1, **characterized in that** the film hinges (13, 14) are formed over the entire length of the end edges of the gripping surfaces (5, 6).

3. The manually operated dispenser as claimed in claim 1 or 2, **characterized in that** the wings (11, 12) in each case have at least one rib (17, 18) running transverse with respect to the film hinges (13, 14) which has a supporting surface (19), which, with the wings (11, 12) unfolded, can be brought into engagement with a stop (20) arranged on the pump head (2).

4. The manually operated dispenser as claimed in one of claims 1 to 3, **characterized in that** the wings (11, 12) each have a blocking wedge (21, 22) which, with the wings (11, 12) folded shut, deflect locking pins (23, 24) arranged on the pump head (2).

5. The manually operated dispenser as claimed in one of claims 1 to 4, **characterized in that** the wings (11, 12) can be fixed in a folded-shut state via a snap-type fastener (27).

6. The manually operated dispenser as claimed in one of claims 1 to 5, **characterized in that** the wings (11, 12) have at least one holding pin (30, 31) projecting with respect to a wing bearing surface (28, 29) for aligning engagement in the respectively other wing (11, 12).

7. The manually operated dispenser as claimed in one of claims 1 to 6, **characterized in that** the gripping surfaces (5, 6) bear protruding anti-slip elements (32) which, when the protective cap (10) is closed, dip into recesses in the region of a base surface of the wings (11, 12).

## Revendications

1. Distributeur à actionnement manuel pour fluides susceptibles de s'écouler, comportant un réservoir présentant une chambre de réserve et une tête de pompage susceptible d'être fixée à celui-ci, laquelle présente un tronçon d'application avec un adaptateur nasal en forme de tubulure présentant un orifice de sortie réalisé sous forme de buse de pulvérisation, et en saillie latérale de celui-ci des surfaces de préhension, et comportant un capuchon de protection présentant deux ailes rabattables et de forme adaptée pour l'adaptateur nasal, **caractérisé en ce que** les surfaces de préhension (5, 6) présentent sur des côtés opposés des arêtes terminales qui sont reliées par une charnière à film (13, 14) respective d'un seul tenant avec une aile (11, 12) du capuchon de protection (10) et les ailes (11, 12) présentent des surfaces de fond (15, 16) qui recouvrent les surfaces de préhension (5, 6).

2. Distributeur à actionnement manuel selon la revendication 1, **caractérisé en ce que** les charnières à film (13, 14) sont réalisées sur toute la longueur des arêtes d'extrémité des surfaces de préhension (5, 6).

3. Distributeur à actionnement manuel selon la revendication 1 ou 2, **caractérisé en ce que** les ailes (11, 12) présentent chacune au moins une nervure (17, 18) s'étendant transversalement aux charnières à film (13, 14); laquelle possède une surface d'appui (19) qui peut être amenée en engagement dans une butée (20) prévue sur la tête de pompage (2) lorsque les ailes (11, 12) sont déployées.

4. Distributeur à actionnement manuel selon l'une des revendications 1 à 3, **caractérisé en ce que** les ailes (11, 12) présentent chacune une partie de blocage (21, 22) qui dévient des goupilles d'arrêt (23, 24) agencées sur la tête de pompage (2) lorsque les ailes (11, 12) sont rabattues.

5. Distributeur à actionnement manuel selon l'une des revendications 1 à 4, **caractérisé en ce que** les ailes (11, 12) peuvent être fixées dans un état fermé par une fermeture à encliquetage (27).

6. Distributeur à actionnement manuel selon l'une des revendications 1 à 5, **caractérisé en ce que** les ailes (11, 12) présentent au moins une goupille de retenue (30, 31) en saillie par rapport à une surface d'appui d'aile (28, 29) pour s'engager en se dressant dans l'autre aile (11, 12) respective.

7. Distributeur à actionnement manuel selon l'une des revendications 1 à 6, **caractérisé en ce que** les surfaces de préhension (5, 6) portent des éléments antipatinants (32) en saillie qui, lorsque le capuchon de protection (10) est fermé, plongent dans des évidements dans la zone d'une surface de fond des ailes (11, 12).
